Europäisches Patentamt

European Patent Office

Office européen des brevets

(11) Publication number: **0 279 778 B1**

# EUROPEAN PATENT SPECIFICATION

(45) Date of publication of patent specification: **13.04.94**   (51) Int. Cl.⁵: **C12N 1/20,** C12N 9/38, C12P 19/02

(21) Application number: **88810076.5**

(22) Date of filing: **09.02.88**

(54) **New strain belonging to genus Thermus, new beta-galactosidase and process for producing same.**

(30) Priority: **09.02.87 JP 26216/87**

(43) Date of publication of application:
**24.08.88 Bulletin 88/34**

(45) Publication of the grant of the patent:
**13.04.94 Bulletin 94/15**

(84) Designated Contracting States:
**DE FR GB NL**

(56) References cited:

**BIOCHEMICAL JOURNAL, vol. 207, 1982, pages 641-644, The Biochemical Society,GB; R.M. DANIEl et al.: "A correlation between protein thermostability andresistance to proteolysis"**

**BIOTECHNOLOGY AND BIOENGINEERING, vol. 26, 1984, pages 1141-1145, John Wiley& Sons, Inc., New York, US; D.A. COWAN et al.: "Some**

**properties of a beta-galactosidase from an extremely thermophilic bacterium"**

(73) Proprietor: **Research Development Corporation of Japan**
**5-2, Nagatacho 2-chome**
**Chiyoda-ku Tokyo(JP)**

Proprietor: **RIKAGAKU KENKYUSHO**
**2-1 Hirosawa**
**Wako-shi Saitama-ken 351-01(JP)**

Proprietor: **MORINAGA MILK INDUSTRY CO., LTD.**
**33-1, Shiba 5-chome**
**Minato-ku, Tokyo(JP)**

(72) Inventor: **Takase, Mitsunori**
**138-10 Minaminakamaru**
**Oomiya-Shi Saitama 330(JP)**
Inventor: **Horikoshi, Kouki**
**39-8, Sakuradai 4-chome**
**Nerima-ku**
**Tokyo 176(JP)**

(74) Representative: **Kerr, Andrew**
**Postfach 444**
**Finkelerweg 44**
**CH-4144 Arlesheim BL (CH)**

EP 0 279 778 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 96, no. 10, May 1982, page 594, abstract no. 160818h,Columbus, Ohio, US; & JP-A-81 154 991 (CHEMICAL INDUSTRIES CO., LTD) 30-11-1981

ABSTRACTS OF THE ANNUAL MEETING OF THE AMERICAN SOCIETY OF MICROBIOLO-GY, vol.80, 1980, abstract K 103, page 1664, Washington, DC, US; J. JOHNSON: "Geneticregulation of beta-galactosidase synthesis in the extreme thermophile thermusT2"

Hackh's Chemical Dictionary, fourth edition, page 434

## Description

This invention relates to a new strain belonging to genus Thermus which produces a new β-galactosidase, to a new β-galactosidase and to a process for producing the same.

β-galactosidase is an enzyme which decomposes lactose into glucose and galactose, and is used for the production of foods such as lactose-hydrolyzed milk, lactose-hydrolyzed solution, etc. The β-galactosidase widely exists in from animals and plants to microorganisms such as fungus, yeast, bacterium, etc., and enzymes which are now used in the food industry are relatively less thermostable β-galactosidases mainly derived from fungus or yeast. These β-galactosidases are disclosed in the official gazette of Jap. Pat. Publn. No. 24094/1978 (Precedent 1) and the official gazette of Jap. Pat. Appln. Laid-open (Kokai) No. 44287/1977 (Precedent 2).

On the other hand, thermostable β-galactosidases are disclosed in *Biotechnology and bioengineering* [vol. 26, p. 1141 (1984)] (Precedent 3), *Journal of Applied Microbiology* [vol. 2, p. 390 (1980)] (Precedent 4), *Canadian Journal of Microbiology* [vol. 22, p.817 (817)] (Precedent 5), the official gazette of Jap. Pat. Appln. Laid-open (Kokai) No. 154991/1981 (Precedent 6) and *Journal of Bacteriology* [vol. 110. p. 691 (1972)] (Precedent 7). However, these conventional β-galactosidases all have one or more disadvantages such as low thermostability, narrow range of optimum pH and the inhibition of enzymatic action by a reaction product, as will be described later (see Table 2). Thus, the fact is that the conventional β-galactosidases are not put into practical use.

As described above, β-galactosidases now used for the production of foods on an industrial scale are relatively less thermostable enzymes, so that the lactose decomposition treatment is carried out generally at 55°C or less. In addition, milk or lactose solution as a raw material is a preferable nutrition source for bacteria. As the result, the putrefaction owing to the saprophyte contamination during the treatment is a serious problem in the food production. Furthermore, an immobilized β-galactosidase results in its being insufficiently washed and sterilized after the completion of the production, as is also a serious problem in the food production. In order to solve these problems, the treatment of an object by using a thermostable β-galactosidase or the washing and the sterilization at a high temperature at which saparophytes are difficult to proliferate is desired, and the development of industrially applicable thermostable β-galactosidase is strongly demanded.

In order to meet such a demand, the search for the foregoing thermostable galactosidase had been attempted, and various thermostable β-galactosidases were found. However, there is no conventionally obtained thermostable β-galactosidase which has all of enzymological properties described in the following Items (1) to (3) required for the industrial use. That is, there is no thermostable β-galactosidase which attains the level of practical application:

(1) Having sufficient thermostability:

Because the thermocoagulation of milk or defatted milk takes place at 78 to 80°C, it is desired that the enzymatic treatment is carried out at a temperature as higher as possible below said temperature. Thus, the enzyme is required to have a sufficient thermostability in the neighborhood of 70 to 75°C.

(2) Having an optimum pH in the range of neutrality to acidity:

As raw materials to be treated with β-galactosidase, milk, defatted milk, cheese whey, lactose solution, etc. are assumed. These are raw materials in the neutral (pH 6.5) to acidic (pH 4.5) range, and thus are required to have sufficient enzymatic activities in said range.

(3) Low inhibition of enzymatic activity by reaction products:

It is required that the lowering of the enzymatic activity by galactose and glucose as reaction products is small when β-galactosidase acts on lactose.

With an intention of creating a β-galactosidase having the properties described above, the present inventors tried to purely isolate a microorganism producing such a β-galactosidase from nature. As a result of it, they found that a microorganism belonging to genus Thermus as an extreme thermophille produced the β-galactosidase having the properties described above from among a large number of microorganisms producing β-galactosidase, and have completed the present invention.

An object of the present invention lies in providing a new microorgamism producing a new β-galactosidase (hereinafter, referred to as "the present enzyme") having a high thermostability, an optimum pH in neutral to acidic range and an enzymatic activity which is not lowered by reaction products.

Another object of the present invention lies in providing the present enzyme and a process for producing the present enzyme.

That is, our invention contemplates to provide a new strain belonging to genus Thermus characterized by producing a new β-galactosidase having an optimum temperature of 75 to 85°C, optimum pH of 4.5 to 6.5 and an enzymatic activity which is lowered by 10% or less in the presence of 50mM each of galactose and glucose when measured at pH 6.5 and 70°C, a new $\beta$-galactosidase characterized by having an optimum temperature of 75 to 85°C, optimum pH of 4.5 to 6.5 and an enzymatic activity which is lowered by 10% or less in the presence of 50mM each of galactose and glucose when measured at pH 6.5 and 70°C and a new process for producing said new $\beta$-galactosidase characterized by culturing the above said new strain belonging to genus Thermus.

According to the present invention, a $\beta$-galactosidase having a high thermostability, a wide action pH range and showing a low inhibition by reaction products can be obtained. That is, the present enzyme enables the treatment of lactose-contained foods such as milk, defatted milk, cheese whey, lactose solution, etc. at a high temperature at which the putrefaction by saprophytes is difficult to be caused. This effect makes the production process administration at the time of treating the foregoing foods simpler, as is very significant for the food industry.

The invention will now be described in more detail with reference to the accompanying drawings in which:-

Fig. 1 is a graph showing the effect of pH on the present enzyme,

Fig. 2 is a graph showing the effect of temperature on the present enzyme, and

Fig. 3 is a graph showing the inhibition of the present enzyme by reaction products.

(1) Acquisition of Microorganism

The present inventors isolated a large number of microorganisms known to produce $\beta$-galactosidases from soils collected from all parts of the country according to *Bergey's Manual of Systematic Bacteriology* (vol. 1; hereinafter referred to as "the manual"). Said microorganisms were cultured to produce $\beta$-galactosidases, and the produced $\beta$-galactosidases were isolated from media. As a result of studying their physicochemical properties, 3 strains producing the desired $\beta$-galactosidase, i.e., ZK-001, ZK-002 and ZK-003 were isolated. The identification of these strains according to the foregoing manual showed that they were all aerobic and asporogenic bacilli, immobile, gram-negative and catalase-positive and had a growth temperature of 40 to 80°C, an optimum growth temperature of 65 to 75°C and an optimum growth pH around neutrality, wherefrom it was obvious that the strains were microorganisms belonging to genus Thermus. On the other hand, on the basis of the test results of their growth on liquid media containing 2% NaCℓ and their assimilation of sugar, they were judged to be new microorganisms different from the known microorganisms belonging to genus Thermus. The present inventors deposited these strains in Fermentation Research Institute; Agency of Industrial Science and Technology and the strains where deposit numbers were given as follows: FERM BP-1678 (Bikoken-kinki No. 9184) for Thermus sp. ZK-001, FERM BP-1679 (Bikoken-kinki No. 9185) for Thermus sp. ZK-002 and FERM BP-1680 (Bikoken-kinki No. 9186) for Thermus sp. ZK-003. The bacteriological properties of these microorganisms are given in Table 1.

Table 1

| | Thermus sp. ZK-001 | Thermus sp. ZK-002 | Thermus sp. ZK-003 |
|---|---|---|---|
| 1. Morphological properties | | | |
| Shape | bacillus | bacillus | bacillus |
| Size | $0.4\sim0.6\mu m \times 2\sim7\mu m$ | $0.4\sim0.6\mu m \times 2\sim7\mu m$ | $0.4\sim0.6\mu m \times 2\sim7\mu m$ |
| Mobility | − | − | − |
| Gram's stain | − | − | − |
| Flagellum | − | − | − |
| Spore | − | − | − |
| 2. Growth on various media* | | | |
| Agar plate medium | circular, protu-berant, orange | circular, protu-berant, yellow | irregularly circu-alar, flat, pale yellow |
| Agar slant medium | flat, orange | flat, yellow | flat, pale yellow |
| Liquid medium | grown cell mass precipitates | grown cell mass precipitates | grown cell mass precipitates |
| Litmus milk | no change | no change | no change |

EP 0 279 778 B1

| | | | |
|---|---|---|---|
| Liquid medium containing 5% NaC$\ell$ | − | − | − |
| Liquid medium containing 2% NaC$\ell$ | + | + | + |
| 3. Growth pH and temperature | | | |
| Growth pH | 5.5~8.5 | 5.5~8.5 | 5.5~8.5 |
| Growth temperature | 40~80℃ | 40~80℃ | 40~80℃ |
| 4. Biochemical properties | | | |
| Nitrate reduction | + | + | + |
| Denitrification reaction | ± | ± | ± |
| VP test | − | − | − |
| Indole formation | − | − | − |
| Hydrogen sulfide formation | − | − | − |
| Starch hydrolysis | ± | ± | ± |
| Citric acid utilization | − | ± | − |
| Inorganic nitrogen source utilization | + $(NH_4)$ | + $(NH_4)$ | + $(NH_4)$ |
| Coloring matter formation | formed (orange) | formed (yellow) | formed (pale yellow) |

EP 0 279 778 B1

| | Column 1 | Column 2 | Column 3 |
|---|---|---|---|
| Urease | − | − | − |
| Oxidase | + | + | + |
| Catalase | + | + | + |
| O-F test | O | O | O |
| Oxygen requirement | Not grown under anaerobic condition | Not grown under anaerobic condition | Not grown under anaerobic condition |
| Gelatin liquefication | + | − | − |
| 5. Assimilation of sugar | | | |
| L-arabinose | ± | + | − |
| D-xylose | ± | + | − |
| D-glucose | + | + | + |
| D-mannose | + | + | + |
| D-fructose | + | + | + |
| D-galactose | + | + | − |
| Maltose | + | + | + |
| Sucrose | + | + | + |
| Lactose | + | + | − |

7

| | Trehalose | D-sorbit | D-mannite | Inosite | Glycerin | Starch |
|---|---|---|---|---|---|---|
| | + | +∣ | ∣ | ∣ | ∣ | ∣ |
| | + | ∣ | + | + | ∣ | ∣ |
| | + | ∣ | ∣ | ∣ | ∣ | ∣ |

In Table 1, * means that media (pH 7.6) having the following composition were used as essential media, excluding litmus milk:

0.1% yeast extract, 0.1% tryptone, 10.0% inorganic salt solution (containing 1.0g of nitrilotriacetic acid, 0.6g of $CaSO_4 \cdot 2H_2O$, 1.0g of $MgSO_4 \cdot 7H_2O$, 0.08g of NaCℓ, 1.03g of $KNO_3$, 6.89g of $NaNO_3$ and 1.11g of $Na_2HPO_4$ per liter of solution), 1.0% ferric chloride solution (containing 0.28g of $FeCℓ_3 \cdot 6H_2O$ per liter of

solution), 1.0% trace element solution (containing 0.5 m$\ell$ of $H_2SO_4$, 2.2g of $MnSO_4 \cdot H_2O$, 0.5g of $ZnSO_4 \cdot 7H_2O$, 0.5 g of $H_3BO_3$, 0.016g of $CuSO_4 \cdot 5H_2O$, 0.025g of $Na_2MoO_4 \cdot 2H_2O$, and 0.046g of $CoC\ell_2 \cdot 6H_2O$), 0.001% thiamine hydrochloride, 0.001% nicotinamide, 0.001% biotin and 0.001% p-aminobenzoic acid.

(2) Acquisition of the present enzyme

Strains producing the present enzyme are inoculated on media which will be described later and aerobically cultured at 50 to 80°C, preferably at 65 to 75°C for 12 to 48 hours.

The media contain inorganic salts, trace nutrients in addition to a carbon source and a nitrogen source according to demand. As a carbon source, various conventionally known materials can be used. For example, glucose, maltose, sucrose or soluble starch can be enumerated as typical examples. As a nitrogen source, there is also no restriction particularly. For example, organic nitrogen such as yeast extract, peptone, meat extract, corn steep liquor, amino acid solution, soybean meal, etc. or inorganic nitrogen such as ammonium sulfate, urea, ammonium nitrate, ammonium chloride, etc. can be enumerated as cheap and readily available sources. Incidentally, it goes without saying that the organic nitrogen source turns into a carbon source. Furthermore, in addition to such carbon and nitrogen sources as above, it is possible to add various salts in use commonly, e.g. inorganic salts such as magnesium salt, potassium salt, phosphate, iron salt, etc.; vitamin; etc. As a suitable medium, a liquid medium containing 0.2% glucose, 0.2% yeast extract, 0.2% tryptone, 10.0% innorganic salt solution (containing 1.0g of nitrilotriacetic acid, 0.6g of $CaSO_4 \cdot 2H_2O$, 1.0g of $MgSO_4 \cdot 7H_2O$, 0.08g of $NaC\ell$, 1.03g of $KNO_3$, 6.89g of $NaNO_3$ and 1.11g of $Na_2HPO_4$ per liter of solution), 1% ferric chloride solution (containing 0.28g of $FeC\ell_3 \cdot 6H_2O$ per liter of solution), 1.0% trace element solution (containing 0.5m$\ell$ of $H_2SO_4$, 2.2g of $MnSO_4 \cdot H_2O$, 0.5g of $ZnSO_4 \cdot 7H_2O$, 0.5g of $H_3BO_3$, 0.016g of $CuSO_4 \cdot 5H_2O$, 0.025g of $Na_2MoO_4 \cdot 2H_2O$, and 0.046g of $CoC\ell_2 \cdot 6H_2O$), 0.001% thiamine hydrochloride, 0.001% nicotinamide, 0.001% biotin and 0.001% p-aminobenzoic acid and having a pH adjusted to 7.6 with NaOH can be enumerated.

The isolation and the purification of the present enzyme can be carried out, for example, as follows. Cell masses in a culture medium are collected by centrifugation, filtration, etc. and the obtained cell masses are disrupted in a buffer (e.g., 10mM phosphate buffer of pH 7.0) in a usual way and then subjected to the extraction treatment. A supernatant of the resulting extract was applied to chromatography such as usual ion-exchange, gel filtration, etc., thereby obtaining the present enzyme.

A preferable method for acquiring the present enzyme can be exemplified as follows. Thermus sp. ZK-001 is inoculated, for example, into 5$\ell$ of such a medium as above and then aerobically cultured at 70°C for 24 hours. The obtained culture medium is centrifuged at 12,000×g at 20°C for 30 minutes so as to collect cell masses, thereby obtaining 55g of cell mass.

To the obtained cell masses, are added approx. 300 m$\ell$ of phosphate buffer (10mM, pH 7.0) for extraction. Thus treated cell masses are disrupted in a mill so as to extract an enzyme. The obtained extract is centrifuged at 40,000×g for 60 minutes and the resulting supernatant is retained. Then, after extracting a precipitate by adding 200m$\ell$ of the above phosphate buffer for extraction, the obtained extract is centrifuged similarly and the supernatant is retained. Whereby, 480m$\ell$ of crude enzyme extract is obtained including the previously obtained supernatant. This crude enzyme solution is saturated to 60% by adding 187g of ammonium sulate thereto and then let stand overnight so as to form a precipitate. The precipitate is collected by centrifuging thus precipitated crude enzyme solution at 30,000×g for 60 minutes. The collected precipitate is dissolved in approx. 200m$\ell$ of 10mM phosphate buffer (pH 7.0) and then dialyzed against said buffer. After discarding a precipitate in thedialyzed enzyme solution by centrifuging the solution at 40,000×g for 60 minutes, thus treated enzyme solution is loaded onto a column of DEAE-TOYOPEARL equilibrated with 10mM phosphate buffer (pH 7.0). Then, the enzyme is eluted according to the concentration gradient method with 10mM phosphate buffer (pH 7.0) containing 0 to 0.5M NaC$\ell$. The eluted active fractions are collected and concentrated with an ultrafilter, followed by dialyzing overnight using 10mM phosphate buffer (pH 7.0) containing 0.1M NaC$\ell$. Thus treated enzyme solution is subjected to the gel filtration using a column of Sephacryl S-300 equilibrated with 10mM phosphate buffer (pH 7.0) containing 0.1M NaC$\ell$ to isolate 3 active fractions. The collected 3 active fractions are respectively dialyzed overnight using 10mM phosphate buffer (pH 7.0) and then loaded onto columns of Sepharose 4B to which p-aminophenyl-$\beta$-D-thiogalactopyranoside equilibrated with said buffer is covalently bonded. After washing these columns thoroughly with 10mM phosphate buffer (pH 7.0), 0.1M borate buffer is passed through the columns, whereby active fractions are eluted and isolated. Thus obtained 3 active fractions are respectively found to be purified to give single bands in the polyacryamide gel electrophoresis (gel concentration: 7.5%, pH: 9.5). The yield of the obtained enzyme preparation amounts to 3.4mg in total of 3 enzymes and the activity yield thereof reaches 12%. Incidentally, with respect to other 2 strains, the present enzyme can be also obtained in the same manner described above while variously changing their culture conditions.

(3) Properties of the present enzyme

The enzymological properties of the present enzyme produced according to the present process are as follows:

A. Action: Hydrolyzing lactose into galactose and glucose.

B. Substrate specificity:

Hydrolyzing lactose, but not hydrolyzing sucrose, melibiose, raffinose and maltose.

C. Optimum pH and stable pH range:

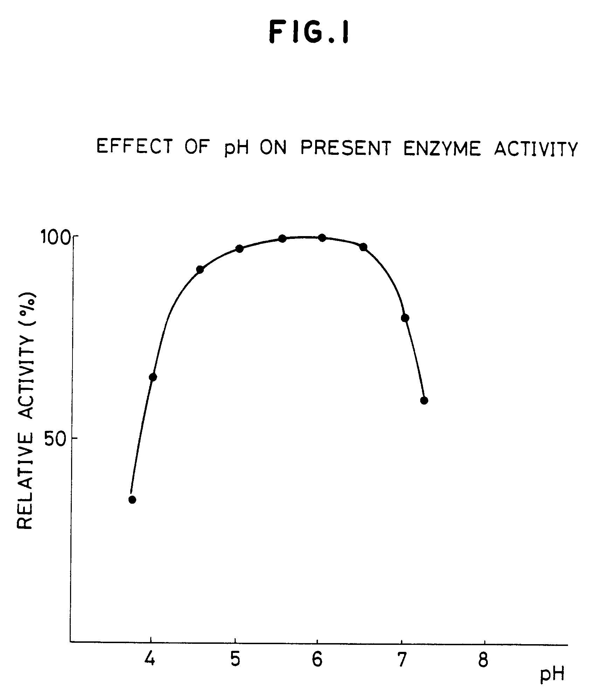

Optimum pH is 4.5 to 6.5. Stable within the pH range of 4.0 to 8.0 under the condition of 24-hour retention at 55°C. (see the graph showing the effect of pH on the present enzyme activity in Fig. 1).

D. Thermostability:

At pH 7.0, 100% activity remains after 1-hour heating at 80°C and 85% activity remains after 1-hour heating at 85°C.

E. Optimum temperature range:

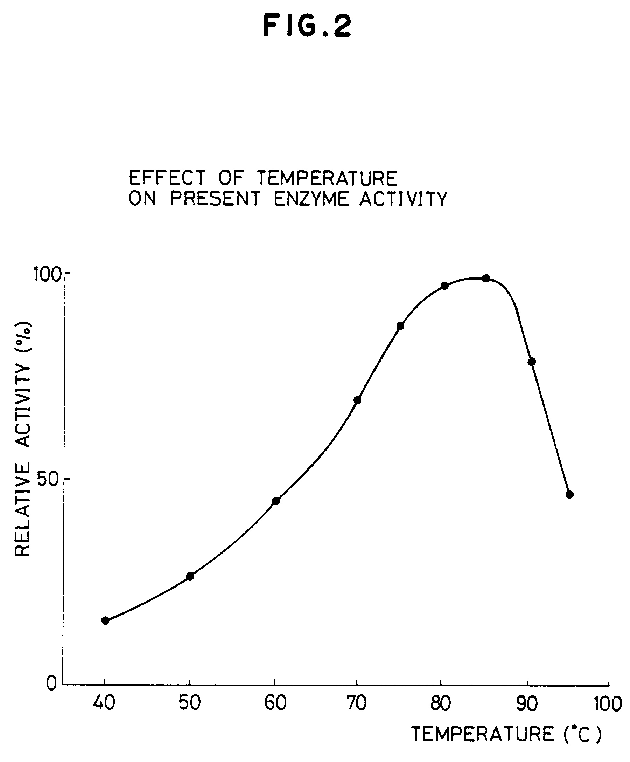

Having an optimum temperature within the range of 75 to 85°C (see the graph showing the effect of temperature on the present enzyme activity in Fig. 2).

F. Effect of inorganic salts:

The enzymatic activity is not changed by 1mM each of ferric chloride, manganese chloride, calcium chloride and magnesium sulfate, but is lowered by 1mM each of zinc chloride and copper sulfate respectively by 10% and 30%.

G. Inhibition by reaction products:

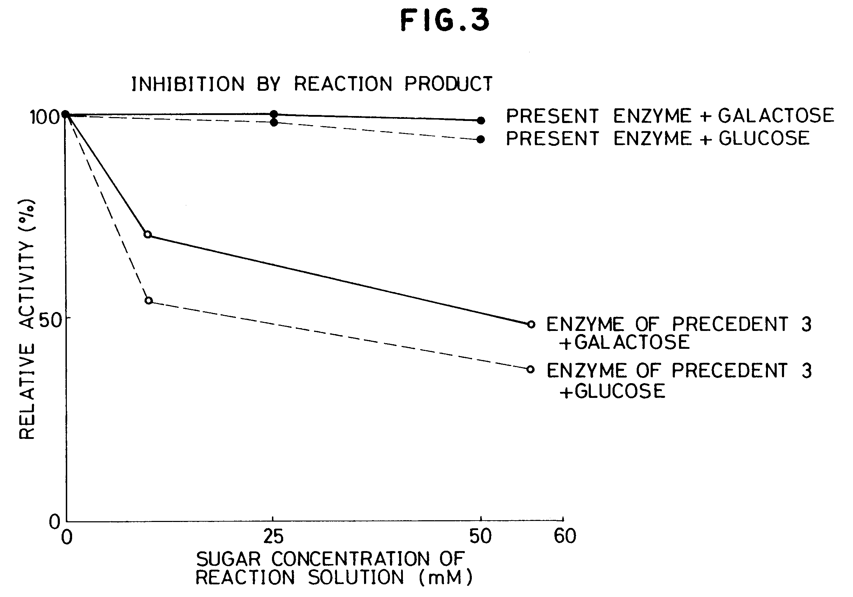

The lowering of the enzymatic activity by 50mM each of galactose and glucose is 10% or less (see the graph showing the inhibition of the present enzyme by reaction product in Fig. 3).

H. Molecular weight: 55,000 daltons.

The determination of molecular weight of the present enzyme according to the gel filtration chromatography using Sephacryl S-300 column (1.6cmx100cm) gives values corresponding to 55,000±5,000 dalton, 110,000±10,000 dalton and 440,000±40,000 dalton.

The physicochemical and enzymological properties of the present enzyme and $\beta$-galactosidase derived from the conventionally known microorganisms (enzymes of the foregoing Precedent 1 to 7) are comparatively given in Table 2.

Incidentally, methods of measuring and indicating the activity are as follows.

That is, 0.1m$\ell$ of the present enzyme solution is added to 2.4m$\ell$ of 0.1M phosphate buffer (pH 6.5) containing 1.50% O-nitrophenyl-$\beta$-D-galactopyranoside (hereinafter referred to as "ONPG") and reacted at 70°C for 10 minutes. Thereafter, the reaction is discontinued by the addition of 2.5m$\ell$ of 10% $Na_2CO_3$ solution. The amount of O-nitrophenol formed is obtained from the absorbance at 420nm, and the amount of enzyme liberating 1$\mu$mol of O-nitrophenol per minute is taken as one unit.

Table 2 - 1

|  | Present enzyme | Enzyme of Precedent 1 | Enzyme of Precedent 2 | Enzyme of Precedent 3 |
|---|---|---|---|---|
| Strain | Thermus sp. | Kluyveromyces lactis | Aspergillus oryzae | Thermus strain 4-1A |
| Optimum temperature of enzymatic reaction | 75~85℃ | 40~50℃ | 50℃ | Not described |
| Thermo-stability of enzyme | 15% inactivation at 85℃ for 1 hr.; no inactivation at 80℃ for 1 hr. | 45% inactivation at 50℃ for 10 min. and 100% in-activation at 55℃ for 10 min. | 50% inactivation at 50℃ for 15 min. | 72% inactivation at 85℃ for 20 hr. and 25% inactivation at 75℃ for 20 min. |
| Optimum pH of enzymatic reaction | pH 4.5 ~ pH 6.5 | pH 6 ~ pH 7 | pH 4.5 | pH 6.0 |
| Inhibition by reaction product | 10% or less inhibition by 50mM each of galactose and glucose | Not described | Not described | 52% inhibition by 56mM of galactose and 63% inhibition by 56mM of glucose |
| Molecular Weight (dalton) | 55,000±5,000 | Not described | Not described | 440,000 |

Table 2 - 2

| | Enzyme of Precedent 4 | Enzyme of Precedent 5 | Enzyme of Precedent 6 | Enzyme of Precedent 7 |
|---|---|---|---|---|
| Strain | Caldarilla acidophila | Bacillus stearothermophilus | Thermus thermophilus | Thermus aquaticus |
| Optimum temperature of enzymatic reaction | 80°C | 65°C | 75~85°C | 80°C |
| Thermostability of enzyme | 50% inactivation at 85°C for 55 hr. and 50% inactivation at 70°C for 55 hr. | 60% inactivation at 60°C for 10 min. | 20% inactivation at 90°C for 20 min. and 5% inactivation at 85°C for 20 min.; no inactivation at 80°C for 150 min. | 55% inactivation at 90°C for 30 min. and 28% inactivation at 80°C for 30 min. |
| Optimum pH of enzymatic reaction | pH 5.0 | pH 6.0 ~ pH 6.4 | pH 5.5 ~ pH 6.5 | pH 5.0 |
| Inhibition by reaction product | No inhibition by 100mM each of galactose and glucose | 20% inhibition by 10mM of glucose and 71% inhibition by 10mM of galactose | Not described | Not described |
| Molecular Weight (dalton) | Not described | 215,000 | 85,000±5,000 | 570,000 |

As shown in Table 2, the enzymological and physicochemical properties of the present enzyme are different from those of all the known β-galactosidases. The present enzyme unites properties of particularly high thermostability, wide optimum pH range and low inhibition by reaction products. That is, the present enzyme posseses excellent properties which the conventional enzymes do not possess. Thus, the present enzyme is suitable for the high temperature processing of various lactose-contained foods such as milk, defatted milk, cheese whey, lactose solution, etc., and thus is industrially useful β-galactosidase.

Hereinafter, the present invention will be described more specifically, referring to examples. However, the present invention is nowise restricted to the examples given below.

Example 1

Thermus sp. ZK-001 (FERM BP-1678) was inoculated into 10ℓ of liquid medium containing 0.2% glucose, 0.2% yeast extract, 0.2% tryptone, 10.0% inorganic salt solution (containing 1.0g of nitrilotriacetic

12

acid, 0.6g of $CaSO_4 \cdot 2H_2O$, 1.0g of $MgSO_4 \cdot 7H_2O$, 0.08g of $NaC\ell$, 1.03g of $KNO_3$, 6.89g of $NaNO_3$ and 1.11g of $Na_2HPO_4$ per liter of solution), 1% ferric chloride solution (containing 0.28g of $FeC\ell_3 \cdot 6H_2O$ pr liter of solution), 1.0% trace element solution (containing $0.5m\ell$ of $H_2SO_4$, 2.2g of $MnSO_4 \cdot H_2O$, 0.5g of $ZnSO_4 \cdot 7H_2O$, 0.5g of $H_3BO_3$, 0.016g of $CuSO_4 \cdot 5H_2O$, 0.025g of $Na_2MoO_4 \cdot 2H_2O$, and 0.046g of $CoC\ell_2 \cdot 6H_2O$), 0.001% thiamine hydrochloride, 0.001% nicotinamide, 0.001% biotin and 0.001% p-aminobenzoic acid and having a pH adjusted to 7.6 with NaOH, and then cultured under aeration in a 20-$\ell$ jar fermentor at 70°C for 24 hours. The enzymatic acitvity of the culture solution was 0.84 unit/m$\ell$.

This culture solution was centrifuged at 12,000×g at 20°C for 30 minutes so as to collect cell masses, thereby obtaining 107g of cell mass. To the obtained cell masses, were added approx. 600m$\ell$ of phosphate buffer (10mM, pH 7.0) for extraction. Thus treated cell masses were disrupted in a mill so as to extract an enzyme. The extract was centrifuged at 40,000×g for 60 minutes and the resulting supernatant was retained. Then, after extracting a precipitate by adding 400m$\ell$ of the above phosphate buffer for extraction, the extract was centrifuged similarly and the supernatant was retained. Whereby, 975m$\ell$ of crude enzyme extract was obtained including the previously obtained supernatant. This crude enzyme solution was saturated to 60% by adding 380g of ammonium sulfate thereto and let stand overnight so as to form a precipitate. Then, the precipitate was collected by the centrifugation at 30,000×g for 60 minutes. The collected precipitate was dissolved in approx. 30m$\ell$ of 10mM phosphate buffer (pH 7.0) and then dialyzed against said buffer. After eliminating a precipitate in the dialyzed enzyme solution by centrifuging the enzyme solution at 40,000×g for 60 minutes, the resulting enzyme solution was loaded onto a column (5cm×45cm) of DEAE-TOYOPEARL equilibrated with 10mM phosphate buffer (pH 7.0). Then, the enzyme was eluted according to the concentration gradient method with 10mM phosphate buffer (pH 7.0) containing 0 to 0.5M NaC$\ell$. 48.0m$\ell$ of eluted active fraction was concentrated to approx. 10m$\ell$ by using an ultrafilter (PM-10 manufactured by Amicon Corp.) and the resulting concentrate was dialyzed overnight using 10mM phosphate buffer (pH 7.0) containing 0.1M NaC$\ell$. Thus treated enzyme solution was passed through a gel filtration column (2.6cm×95cm) of Sephacryl S-300 equilibrated with 10mM phosphate buffer (pH 7.0) containing 0.1M NaC$\ell$ to isolate 3 active fractions. Said 3 fractions were combined, dialyzed overnight using 10mM phosphate buffer, and then loaded onto a column of Sepharose 4B to which p-aminophenyl-$\beta$-D-thiogalactopyranoside equilibrated with said buffer was covalently bonded. After washing this column thoroughly with 10mM phosphate buffer (pH 7.0), 0.1M borate buffer was passed through the column to elute and isolate active fractions. As a result, approx. 8.5mg of the present enzyme was obtained.

Example 2

Thermus sp. ZK-002 was inoculated into 10$\ell$ of the same medium as in Example 1, except that 0.2% soluble starch was used instead of glucose. The culture was carried out under the same conditions as in Example 1, whereby 0.64 units/m$\ell$ of culture solution was obtained. Hereinafter, the purification was carried out in the same manner as in Example 1, whereby approx. 6.8mg of the present enzyme was obtained.

Example 3

Thermus sp. ZK-003 was inoculated into 10$\ell$ of medium having the same composition as discribed in Example 1 and cultured under the same conditions as in example 1, whereby 0.68 units/m$\ell$ of culture solution was obtained. Hereinafter, the purification was carried out in the same manner as in Example 1, whereby approx. 7.3mg of the present enzyme was obtained.

In this specification and the claims the symbol "mM" means " millimoles per litre".

**Claims**

1. A new strain belonging to genus Thermus, characterized by producing a new $\beta$-galactosidase having an optimum temperature of 75 to 85°C, an optimum pH of 4.5 to 6.5 and an enzymatic activity which is lowered by 10% or less in the presence of 50mM each of galactose and glucose when measured at pH 6.5 and 70°C.

2. A new strain belonging to genus Thermus according to Claim 1, wherein the new strain has at least the following bacteriological properties:
    A. Morphological properties:

EP 0 279 778 B1

| Shape | bacillus |
|---|---|
| Mobility | - |
| Gram's stain | - |
| Flagellum | - |
| Spore | - |
| Acid-fastness | - |
| Size | 0.4~0.6um x 2~7um |

B. Growth on various media:

| Agar plate medium | circular to irregularly circular, protuberant to flat, pale yellow to orange |
|---|---|
| Agar slant medium | smooth, pale yellow to orange |
| Liquid medium | grown cell mass precipitates |
| Litmus milk | no change |
| Liquid medium containing 5% NaCl | - |
| Liquid medium containing 2% NaCl | + |

C. Growth pH and temperature:

| Growth pH | 5.5~8.5 |
|---|---|
| Growth temperature | 40~80 °C |

D. Biochemical properties:

| Nitrate reduction | + |
|---|---|
| Denitrification reaction | ± |
| VP test | - |
| Indole formation | - |
| Hydrogen sulfide formation | - |
| Starch hydrolysis | ± |
| Citric acid utilization | - |
| Inorganic nitrogen source utilization | + (NH$_4$) |
| Coloring matter formation formed (yellow, orange) | |
| Urease | - |
| Oxidase | + |
| Catalase | + |
| O-F test | O |
| Oxygen requirement | Not grown under anaerobic condition |

E. Assimilation of sugar:

| D-glucose | + |
|---|---|
| D-mannose | + |
| D-fructose | + |
| maltose | + |
| sucrose | + |
| lactose | + |
| trehalose | + |
| glycerin | - |
| starch | - |

14

3. A new strain belonging to genus Thermus according to Claim 1 or 2, wherein the new strain is selected from the group consisting of FERM BP-1678 (Bikoken-kinki No. 9184), FERM BP-1679 (Bikoken-kinki No. 9185) and FERM BP-1680 (Bikoken-kinki No. 9186).

4. A new $\beta$-galactosidase, characterized by having an optimum temperature of 75 to 85°C, an optimum pH of 4.5 to 6.5 and an enzymatic activity which is lowered by 10% or less in the presence of 50mM each of galactose and glucose when measured at pH 6.5 and 70°C.

5. A new $\beta$-galactosidase according to Claim 1, wherein the new $\beta$-galactosidase has the following physicochemical properties:
   A. Action: Having an action of hydrolyzing lactose into galactose and glucose.
   B. Substrate specificity:
      Hydrolyzing lactose but not hydrolyzing sucrose, melibiose, raffinose and maltose.
   C. Optimum pH and stable pH range:
      Optimum pH is 4.5 to 6.5. Stable within the pH range of 4.0 to 8.0 under the condition of 24-hour retention at 55°C.
   D. Thermostability:
      At pH 7.0, 100% enzymatic activity remains after 1-hour heating at 80°C and 85% enzymatic activity remains after 1-hour heating at 85°C.
   E. Optimum temperature range:
      Having an optimum temperature within the range of 75 to 85°C.
   F. Effect of inorganic salts:
      The enzymatic activity is not changed by 1mM each of ferric chloride, manganese chloride, calcium chloride and magnesium sulfate, but is lowered by 1mM each of zinc chloride and copper sulfate respectively by 10% and 30%.
   G. Inhibition by reaction products:
      The lowering of the enzymatic activity by 50mM each of galactose and glucose is 10% or less respectively.
   H. Molecular weight: 55,000 daltons.

6. A process for producing a new $\beta$-galactosidase, characterized by culturing a strain belonging to genus Thermus which produces a new $\beta$-galactosidase having an optimum temperature of 75 to 85°C, an optimum pH of 4.5 to 6.5 and an enzymatic activity which is lowered by 10% or less in the presence of 50mM each of galactose and glucose when measured at pH 6.5 and 70°C, and collecting the produced $\beta$-galactosidase.

7. A process according to Claim 6, wherein the culture is carried out aerobically at a temperature of 50 to 80°C.

8. A process according to Claims 6 or 7, wherein the culture is carried out at a pH of 6 to 8.5.

9. A process according to Claims 6, 7 or 8, wherein the strain belonging to genus Thermus is FERM BP-1678 (Bikoken-kinki No. 9184), FERM BP-1679 (Bikoken-kinki No. 9185) or FERM BP-1680 (Bikoken-kinki No. 9186) or their mixture.

10. A process for the production of galactose and glucose from lactose, which comprises using a microorganism as claimed in any one of Claims 1 to 3 or a $\beta$-galactosidase prepared by a process as claimed in any one of Claims 6 to 9.

**Patentansprüche**

1. Neuer Stamm der Gattung Thermus, gekennzeichnet durch die Produktion einer neuen $\beta$-Galactosidase mit einem Temperaturoptimum von 75 bis 85°C, einem pH-Optimum von 4,5 bis 6,5 und einer Enzymaktivität, die in Gegenwart von jeweils 50 mM Galactose und Glucose um 10 % oder weniger erniedrigt ist, wenn die Messung bei pH 6,5 und 70°C erfolgt.

2. Neuer Stamm der Gattung Thermus nach Anspruch 1, wobei der neue Stamm mindestens die folgenden bakteriologischen Eigenschaften aufweist:

15

A. Morphologische Eigenschaften:

| Form | Bacillus |
|---|---|
| Mobilität | - |
| Gramfärbung | - |
| Flagellum | - |
| Spore | - |
| Säurebeständigkeit | - |
| Größe | 0,4-0,6 $\mu$m x 2-7 $\mu$m |

B. Wachstum auf verschiedenen Medien:

| Agarplattenmedium | zirkulär bis unregelmäßig zirkulär, hervorstehend bis flach, blaßgelb bis orange |
|---|---|
| Agarschrägmedium | glatt, blaßgelb bis orange |
| flüssiges Medium | gewachsene Zellmasse fällt aus |
| Lackmusmilch | keine Änderung |
| 5 % NaCl enthaltendes flüssiges Medium | - |
| 2 % NaCl enthaltendes flüssiges Medium | + |

C. Wachstums-pH und -temperatur:

| Wachstums-pH | 5,5~8,5 |
|---|---|
| Wachstumstemperatur | 40~80 °C |

D. Biochemische Eigenschaften:

| Nitratreduktion | + |
|---|---|
| Denitrifizierungsreaktion | ± |
| VP-Test | - |
| Indolbildung | - |
| Schwefelwasserstoffbildung | - |
| Stärkehydrolyse | ± |
| Citronensäureverwertung | - |
| Anorganische Stickstoffquellenverwertung | + → ($NH_4$) |
| Farbstoffbildung | gebildet (gelb, orange) |
| Urease | - |
| Oxidase | + |
| Katalase | + |
| O-F-Test | 0 |
| Sauerstofferfordernis | Wächst nicht unter anaeroben Bedingungen |

E. Assimilation von Zucker:

| | |
|---|---|
| D-Glucose | + |
| D-Mannose | + |
| D-Fructose | + |
| Maltose | + |
| Saccharose | + |
| Lactose | + |
| Trehalose | + |
| Glycerin | - |
| Stärke | - |

3. Neuer Stamm der Gattung Thermus nach Anspruch 1 oder 2, wobei der neue Stamm ausgewählt ist aus FERM BP-1678 (Bikoken-kinki Nr. 9184), FERM BP-1679 (Bikoken-kinki Nr. 9185) und FERM BP-1680 (Bikoken-kinki Nr. 9186).

4. Neue β-Galactosidase, dadurch gekennzeichnet, daß sie ein Temperaturoptimum von 75 bis 85°C, ein pH-Optimum von 4,5 bis 6,5 und eine Enzymaktivität aufweist, die in Gegenwart von jeweils 50 mM Galactose und Glucose um 10 % oder weniger erniedrigt ist, wenn die Messung bei pH 6,5 und 70°C erfolgt.

5. Neue β-Galactosidase nach Anspruch 1, wobei die neue β-Galactosidase die folgenden physikalisch-chemischen Eigenschaften aufweist:
   A. Wirkung:
   hydrolysiert Lactose in Galactose und Glucose.
   B. Substratspezifität:
   hydrolysiert Lactose, aber nicht Saccharose, Melibiose, Raffinose und Maltose.
   C. pH-Optimum und pH-Stabilitätsbereich:
   pH-Optimum bei 4,5 bis 6,5. Stabil in einem pH-Bereich von 4,0 bis 8,0 über einen Zeitraum von 24 Stunden bei 55°C.
   D. Hitzestabilität:
   Bei pH 7,0 bleiben 100 % der Enzymaktivität nach 1stündigem Erhitzen auf 80°C und 85 % der Enzymaktivität nach 1stündigem Erhitzen auf 85°C erhalten.
   E. Temperaturoptimumbereich:
   Temperaturoptimum in einem Bereich von 75 bis 85°C.
   F. Wirkung von anorganischen Salzen:
   Die Enzymaktivität verändert sich nicht durch 1 mM Eisen(III)-chlorid, Manganchlorid, Calciumchlorid und Magnesiumsulfat, aber wird durch 1 mM Zinkchlorid und Kupfersulfat um 10 % bzw. um 30 % erniedrigt.
   G. Hemmung durch Reaktionsprodukte:
   Die Erniedrigung der Enzymaktivität durch 50 mM Galactose bzw. Glucose beträgt 10 % oder weniger.
   H. Molekulargewicht: 55 000 Daltons.

6. Verfahren zur Herstellung einer neuen β-Galactosidase, gekennzeichnet durch die Züchtung eines Stammes der Gattung Thermus, der eine neue β-Galactosidase produziert mit einem Temperaturoptimum von 75 bis 85°C, einem pH-Optimum von 4,5 bis 6,5 und einer Enzymaktivität, die in Gegenwart von jeweils 50 mM Galactose und Glucose um 10 % oder weniger erniedrigt ist, wenn die Messung bei pH 6,5 und 70°C erfolgt, und Gewinnung der produzierten β-Galactosidase.

7. Verfahren nach Anspruch 6, wobei die Züchtung aerob bei einer Temperatur von 50 bis 80°C durchgeführt wird.

8. Verfahren nach Anspruch 6 oder 7, wobei die Züchtung bei einem pH-Wert von 6 bis 8,5 durchgeführt wird.

9. Verfahren nach Anspruch 6, 7 oder 8, wobei der Stamm der Gattung Thermus FERM BP-1678 (Bikoken-kinki Nr. 9184), FERM BP-1679 (Bikoken-kinki Nr. 9185) oder FERM BP-16808 (Bikoken-kinki

EP 0 279 778 B1

Nr. 9186) oder deren Gemisch ist.

10. Verfahren zur Produktion von Galactose und Glucose aus Lactose, umfassend die Verwendung eines Mikroorganismus nach einem der Ansprüche 1 bis 3 oder einer $\beta$-Galactosidase, die gemäß einem Verfahren nach einem der Ansprüche 6 bis 9 hergestellt wurde.

**Revendications**

1. Nouvelle souche appartenant au gène Thermus, caractérisée par la production d'une nouvelle $\beta$-galactosidase ayant une température optimale de 75 à 85°C, un pH optimum de 4,5 à 6,5 et une activité enzymatique qui est diminuée de 10% ou moins en présence de 50mM chacun de galactose et glucose lorsque mesurée à pH 6,5 et 70°C.

2. Nouvelle souche appartenant au gène Thermus selon la revendication 1, dans laquelle la nouvelle souche a au moins les propriétés bactériologiques suivantes :
A. Propriétés morphologiques :

| Forme | bacillus |
|---|---|
| Mobilité | - |
| Coloration de Gram | - |
| Flagelle | - |
| Spore | - |
| Résistance aux acides | - |
| Dimension | 0,4~0,6μm x 2~7μm |

B. Croissance sur des milieux variés :

| Milieu plaque agar | circulaire à irrégulièrement circulaire, protubérant à plat, jaune pâle à orange |
|---|---|
| Milieu gélose en pente | lisse, jaune pâle à orange |
| Milieu liquide | précipités de masse de cellules grandies |
| Lait de tournesol | aucun changement |
| Milieu liquide contenant 5% de NaCl | - |
| Milieu liquide contenant 2% de NaCl | + |

C. pH et température de croissance :

| pH de croissance | 5,5~8,5 |
|---|---|
| Température de croissance | 40~80°C |

D. Propriétés biochimiques :

18

| Réduction de nitrate | + |
|---|---|
| Réaction de dénitrification | ± |
| Test VP | - |
| Formation d'indole | - |
| Formation de sulfure d'hydrogène | - |
| Hydrolyse d'amidon | ± |
| Utilisation d'acide citrique | - |
| Utilisation d'une source d'azote inorganique | + (NH$_4$) |
| Formation de matières colorées | formées (jaune, orange) |
| Uréase | - |
| Oxydase | + |
| Catalase | + |
| Test 0-F | 0 |
| Exigence en oxygène | Ne croît pas en condition anaérobie |

E. Assimilation du sucre :

| D-glucose | + |
|---|---|
| D-mannose | + |
| D-fructose | + |
| maltose | + |
| sucrose | + |
| lactose | + |
| tréhalose | + |
| glycérine | - |
| amidon | - |

3.  Nouvelle souche appartenant au gène Thermus selon la revendication 1 ou 2, dans laquelle la nouvelle souche est sélectionnée parmi le groupe consistant du FERM BP-1678 (Bikoken-kinki N° 9184), FERM BP-1679 (Bikoken-kinki N° 9185) et FERM BP-1680 (Bikoken-kinki N° 9186).

4.  Nouvelle $\beta$-galactosidase, caractérisée en ce qu'elle a une température optimale de 75 à 85°C, un pH optimum de 4,5 à 6,5 et une activité enzymatique qui est abaissée de 10% ou moins en présence de 50 mM chacun de galactose et glucose lorsque mesurée à pH 6,5 et 70°C.

5.  Nouvelle $\beta$-galactosidase selon la revendication 1, dans laquelle la nouvelle $\beta$-galactosidase a les propriétés physicochimiques suivantes :
    A. Action : ayant une action d'hydrolyse de lactose en galactose et glucose.
    B. Spécificité de substrat :
        Hydrolyse le lactose mais n'hydrolyse pas le sucrose, le mélibiose, le raffinose et le maltose.
    C. pH optimum et intervalle de pH stable :
        le pH optimum est de 4,5 à 6,5. Stable dans l'intervalle de pH de 4,0 à 8,0 sous la condition de rétention de 24 heures à 55°C.
    D. Thermostabilité :
        A pH 7,0, une activité enzymatique de 100% reste après 1 heure de chauffage à 80°C et une activité enzymatique de 85% reste après 1 heure de chauffage à 85°C.
    E. Intervalle de température optimale :
        A une température optimale dans l'intervalle de 75 à 85°C.
    F. Effet des sels inorganiques :
        L'activité enzymatique n'est pas changée par 1mM de chacun du chlorure ferrique, chlorure de manganèse, chlorure de calcium et sulfate de magnésium, mais est abaissée par 1mM chacun du chlorure de zinc et sulfate de cuivre respectivement de 10% et 30%.
    G. Inhibition par les produits de réaction :
        L'abaissement de l'activité enzymatique par 50mM chacun de galactose et glucose est de 10% ou moins respectivement.

H. Poids moléculaire : 55000 daltons.

6. Procédé pour la production d'une nouvelle *β*-galactosidase, caractérisé par la culture d'une souche appartenant au gène Thermus qui produit une nouvelle *β*-galactosidase ayant une température optimale de 75 à 85°C, un pH optimum de 4,5 à 6,5 et une activité enzymatique qui est abaissée de 10% ou moins en présence de 50mM chacun de galactose et glucose lorsque mesurée à pH 6,5 et 70°C, et par la collecte de la *β*-galactosidase produite.

7. Procédé selon la revendication 6, dans lequel la culture est mise en oeuvre de façon aérobie à une température de 50 à 80°C.

8. Procédé selon la revendication 6 ou 7, dans lequel la culture est mise en oeuvre à un pH de 6 à 8,5.

9. Procédé selon les revendications 6, 7 ou 8, dans lequel la souche appartenant au gène Thermus et le FERM BP-1678 (Bikoken-kinki N° 9184), FERM BP-1679 (Bikoken-kinki N° 9185) ou FERM BP-1680 (Bikoken-kinki N° 9186) ou leur mélange.

10. Procédé pour la production de galactose et glucose à partir de lactose, qui comprend l'utilisation d'un microorganisme tel que revendiqué dans l'une quelconque des revendications 1 à 3 ou d'une *β*-galactosidase préparée par un procédé selon l'une quelconque des revendications 6 à 9.

# FIG.I

EFFECT OF pH ON PRESENT ENZYME ACTIVITY

# FIG.2

EFFECT OF TEMPERATURE
ON PRESENT ENZYME ACTIVITY

FIG.3

INHIBITION BY REACTION PRODUCT

PRESENT ENZYME + GALACTOSE
PRESENT ENZYME + GLUCOSE

ENZYME OF PRECEDENT 3 +GALACTOSE

ENZYME OF PRECEDENT 3 +GLUCOSE

RELATIVE ACTIVITY (%)

SUGAR CONCENTRATION OF REACTION SOLUTION (mM)

EP 0 279 778 B1